(19) Europäisches Patentamt
European Patent Office
Office européen des brevets

(11) EP 0 330 240 B1

(12) **EUROPÄISCHE PATENTSCHRIFT**

(45) Veröffentlichungstag und Bekanntmachung des Hinweises auf die Patenterteilung:
**15.01.2003 Patentblatt 2003/03**

(51) Int Cl.[7]: **A61K 35/78**

(21) Anmeldenummer: **89103429.0**

(22) Anmeldetag: **27.02.1989**

(54) **Kamillendroge und ihre Verwendung zur Herstellung von Kamillenextrakten, Kamillenöl und pharmazeutischen Mitteln**

Camomile drug and its use in the preparation of camomile extracts, camomile oil and pharmaceutical agents

Médicament à base de camomille et son application à la préparation d'extraits de camomille, d'huile de camomille et de moyens pharmaceutiques

(84) Benannte Vertragsstaaten:
**AT BE CH DE FR GB IT LI LU NL**

(30) Priorität: **26.02.1988 DE 3806210**

(43) Veröffentlichungstag der Anmeldung:
**30.08.1989 Patentblatt 1989/35**

(73) Patentinhaber: **ROBUGEN GMBH PHARMAZEUTISCHE FABRIK**
**73730 Esslingen (DE)**

(72) Erfinder:
- **Hölzl, Josef, Prof. Dr.**
  **D-3550 Marburg (DE)**
- **Berndt, Dieter, Dipl.-Kaufmann**
  **D-7300 Esslingen-Zell (DE)**
- **Hempel, Bernd, Dr.**
  **D-7300 Esslingen-Zell (DE)**

(74) Vertreter: **Kinzebach, Werner, Dr.**
**Patentanwälte**
**Reitstötter, Kinzebach und Partner**
**Postfach 86 06 49**
**81633 München (DE)**

(56) Entgegenhaltungen:

| | |
|---|---|
| **EP-A- 0 154 872** | **DE-A- 2 402 802** |
| **FR-A- 2 547 982** | **GB-A- 1 560 371** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft eine Kamillendroge aus einer neuen tetraploiden Kamillensorte und die Verwendung dieser Droge zur Herstellung von Kamillenextrakten, Kamillenöl und pharmazeutischen Mitteln.

Die Kamille (Matricaria chamomilla L) ist eine altbekannte und sehr häufig verwendete Heilpflanze. Insbesondere die aus den Blütenköpfchen der Kamille hergestellten pharmazeutischen Mittel finden eine breite therapeutische Anwendung, da sie Wirkstoffe mit antiphlogistischer und spasmolytischer Wirkung enthalten.

Kamillen-typische Inhaltsstoffe sind z.B.:

Farnesen, Chamazulen, En-In-Dicycloether (liegt in trans- und cis-Form vor) , (-)-α-Bisabolol, (-)-α-Bisabololoxid A, (-)-α-Bisabololoxid B und α-Bisabolonoxid. Die Strukturformeln dieser Substanzen sind nachstehend wiedergegeben:

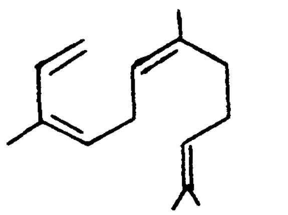

Farnesen (α-Form)

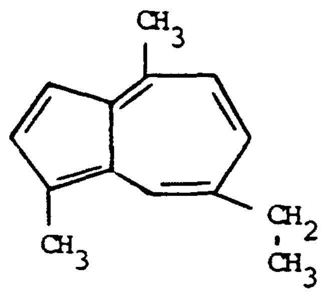

Chamazulen

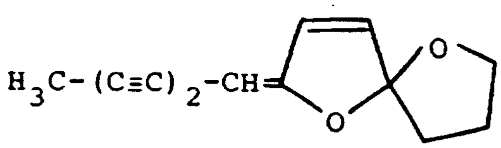

En-In-Dicycloether (cis bzw. trans)

Es besteht folgende strukturelle Beziehung zwischen den Bisaboloiden im etherischen Öl der Kamille.

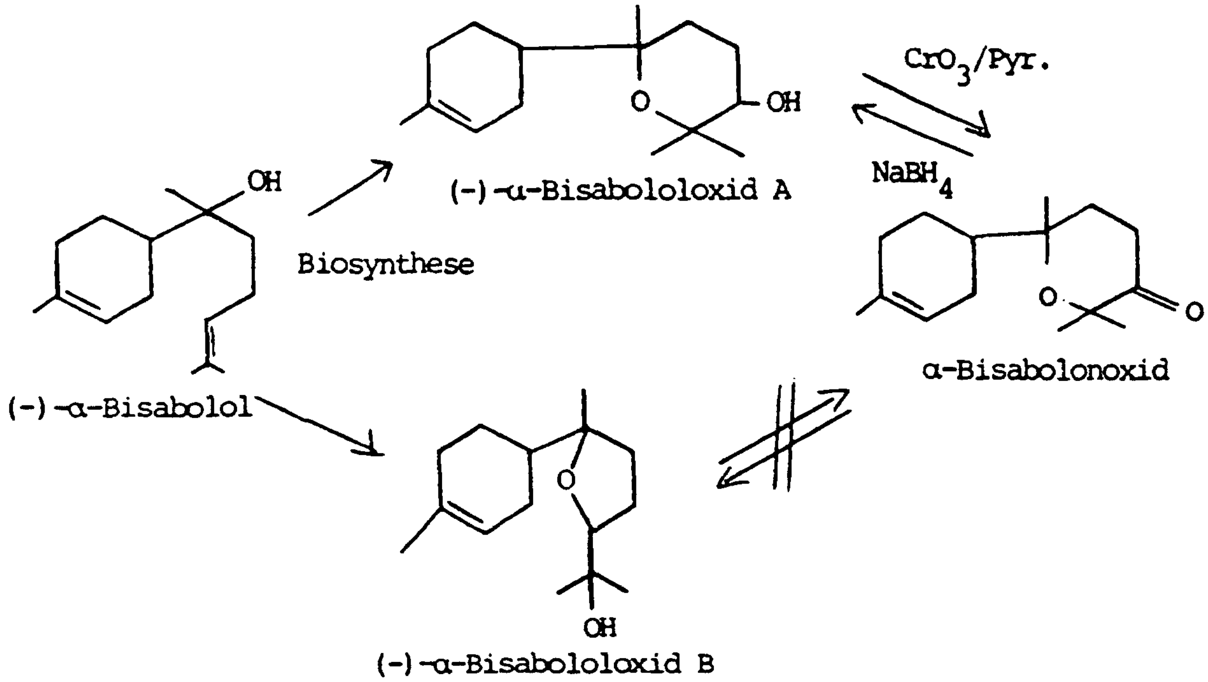


(-)-α-Bisabololoxid A

α-Bisabolonoxid

(-)-α-Bisabolol

(-)-α-Bisabololoxid B

Insbesondere dem Chamazulen und (-)-α-Bisabolol werden antiphlogistische Wirkung zugeschrieben.

Weitere Kamilleninhaltsstoffe sind die Flavonoide, die sich primär in den Zungenblüten der Kamillenblüten befinden. Dazu zählen Apigenin und Apigenin-7-glucosid der Formeln:

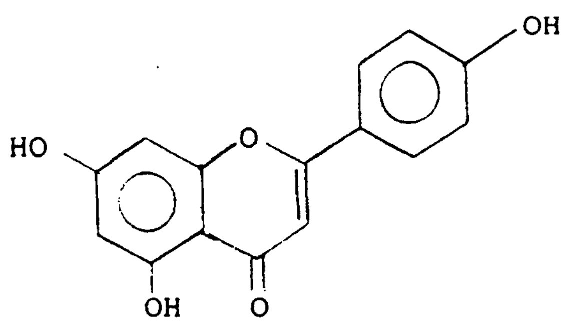

Apigenin

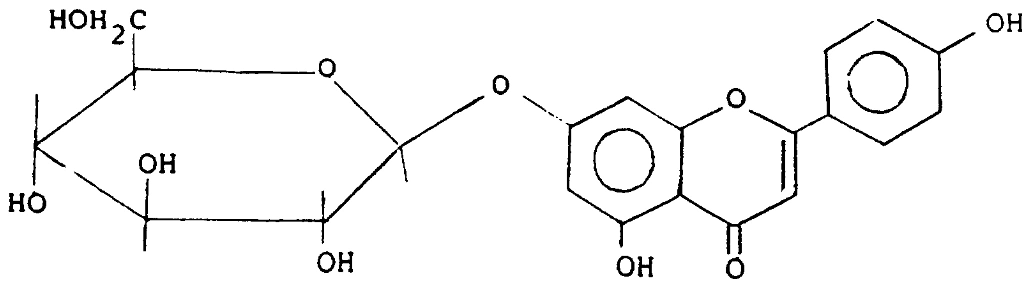

Apigenin-7-glucosid

und dessen Acetylderivate. Nach Untersuchungen von Redaelli u. Mitarb., Planta Medica 42, 288 (1981) sind dies die Hauptflavonoide der Kamille.
Sie sind vor allem für die muskulotrop-spasmolytische Wirkung der Droge verantwortlich. Dabei ist Apigenin muskulotropspasmolytisch wesentlich wirksamer als sein 7-Glucosid und es wirkt antiinflammatorisch.

Es sind bereits verschiedene Kamillensorten mit einem hohen Gehalt an (-)-α-Bisabolol und Chamazulen bekannt. Dazu gehört beispielsweise die in der DE-PS 24 02 802 mit DEGUMILLE bezeichnete Kamillensorte. Sie ist jedoch eine diploide Kamille, bei der aufgrund der Fremdbestäubung durch die überall vorhandene Wildkamille der Wirkstoffgehalt nach zwei- bis dreimaligem Anbau beträchtlich abnimmt.

Es sind auch verschiedene tetraploide Kamillensorten bekannt (z.B. BODEGOLD, DDR; Zloty Lan, Polen).

Bei den bekannten Kamillensorten ist das Apigenin-7-Glucosid mit etwa 100 bis 300 mg/100 g Blüten das dominierende Flavonoid und in wesentlich größeren Anteilen vorhanden als das Apigenin, das nur etwa 5 %, bezogen auf das 7-Glucosid, ausmacht, vgl. z.B. Krankenhauspharmazie, 6, S.405-9, insbes. S. 408, mittlere Spalte, 1985. Apigenin ist jedoch pharmakologisch wesentlich wirksamer und somit auch wertvoller als sein 7-Glucosid.

Es gelang nun überraschenderweise, eine Kamillendroge (Kamillenblüten frisch oder getrocknet) bereitzustellen, die nicht nur einen hohen Gehalt an (-)-α-Bisabolol und Chamazulen, sondern auch einen wesentlich höheren Gehalt an freiem Apigenin aufweist als die bekannten Drogen. In der Regel beträgt der Apigeningehalt der neuen Kamille 15-30 %, bezogen auf den Gehalt an 7-Glucosid und ist damit 3 bis 6 mal so noch wie bei den bekannten Drogen.

Die erfindungsgemäße Kamillendroge ist erhältlich aus einer tetraploiden Kamille ungarischer Herkunft aus dem Institute de Recherche des Plantes Medicinal Danel U. 38 - 42 H - 2011 Budakalascz mit hohem Bisabololoxidgehalt.

Diese Sorte ist eine tetraploide Kamille. Sie ist somit frei von den eingangs geschilderten Nachteilen der bekannten Kamillensorte Degumille.

Gegenstand der Erfindung ist somit Kamillendroge der Kulturpflanzenart echte Kamille (Matricaria chamomilla L) mit einem Chamazulengehalt von 100 bis 250 mg, einem (-)-α-Bisabololgehalt von 150 bis 300 mg, einem Cis-En-In-Dicycloethergehalt von 200 bis 350 mg, einem Gehalt an freiem Apigenin von 30 bis 300 mg, und einem Gehalt an Apigenin-7-glucosid von 150 bis 700 mg, bezogen auf 100 g bei 30° bis 50°C bis zu einem Restfeuchtegehalt von 5 bis 10% getrocknete Kamillenblüten.

Vorzugsweise beträgt der Gehalt an Chamazulen 100 bis 200 mg, insbesondere 100 bis 160 mg;

an (-)-α-Bisabolol 170 bis 250 mg, insbesondere 170 bis 230 mg;
an Cis-En-In-Dicycloether 200 bis 300 mg, insbesondere 200 bis 250 mg;
an freiem Apigenin 40 bis 120 mg, insbesondere 50 bis 100 mg und
an Apigenin-7-glucosid 200 bis 600 mg und insbesondere 250 bis 450 mg.

Gegenstand der Erfindung ist ferner die Verwendung der erfindungsgemäßen Kamillendroge zur Herstellung von Kamillenextrakten, Kamillenöl und pharmazeutischen Mitteln.

Die Kamillenblüten der Erfindung sind vorzugsweise erhältlich aus einer Kamille der Sorte "Robumille".

Diese Blüten haben einen aromatischen, angenehmen, charakteristischen Geruch und einen schwach bitteren Geschmack.

Die Sorte Robumille wurde ausgehend von der oben beschriebenen Kamillensorte wie folgt gezüchtet und auf die wichtigsten Inhaltsstoffe selektiert.

Das Saatgut wurde jedes Jahr im September in Anzuchtkästen oberflächlich ausgesäht und im Gewächshaus mit zusätzlicher Befeuchtung zum Keimen gebracht.

Die Pflanzen waren ca. 14 Tage später aufgelaufen, bei einer Keimfähigkeit von ca. 70 %. Nach Erreichen des Vier- bis Fünfblattstadiums wurden die herangezogenen Jungpflanzen nach gleicher Größe und Form pikiert und in Jiffy-pots eingepflanzt. Die pikierten Pflanzen kamen anschließend für 14 Tage zur Abhärtung ins Kalthaus und Anfang November zur Überwinterung ins Freiland.

Im März des folgenden Jahres wurden 200 Pflanzen in Tontöpfe (Ø 15 cm) mit einem Humus-Sandgemisch von 65:35 (pH 6,5) eingepflanzt. Da für die Selektionsarbeiten blühende Pflanzen für einen längeren Zeitraum benötigt wurden, wurde bereits im April ein Teil der Pflanzen im Warmhaus vorzeitig zum Blühen gebracht.

Zur Überprüfung des Inhaltsstoffmusters und zur Selektion wurde von jeder Pflanze die zuerst aufgeblühte oberste Blüte geerntet. Die Untersuchung erfolgte mittels verschiedener Verfahren.

Zur Untersuchung auf die Inhaltsstoffe Bisabolol, En-In-Dicycloether und Azulen wurden 2 chromatographische Verfahren zur Anwendung gebracht. Der Gehalt an Apigenin und Apigenin-7-glucosid wurde mit Hilfe der Hochdruckflüssigkeitschromatographie bestimmt.

Beim ersten der obengenannten dünnschichtchromatographischen Verfahren wurde ein Teil der Blüte 1 Tag bei 30°C getrocknet, grob zerkleinert und mit Hilfe eines TAS-Ofens (Fa. Desaga, Heidelberg) während eines Zeitraums von 2 Min. bei 220°C auf eine DC-Platte aufgedampft. Die DC-Platte wurde entwickelt, getrocknet, mit Anisaldehyd-Schwefelsäurereagens besprüht und 5 Min. bei 120°C im Trockenschrank erhitzt.

Beim zweiten obengenannten dünnschichtchromatographischen Verfahren wurde ein Teil der geernteten Blüte zerkleinert,im frischen Zustand mit 1 ml Dichlormethan/Methanol (1:1) versetzt und 1/2 h bei Zimmertemperatur geschüttelt. Von dem Extrakt wurden 30µl auf eine DC-Fertigplatte aufgetragen.

| | |
|---|---|
| Schicht: | DC-Fertigplatten-Kieselgel 60 $F_{254}$ (MERCK) 0,25 mm |
| Fließmittel: | Toluol/Ethylacetat (93:7) |
| Detektion: | a) unter UV-Licht (Wellenlänge 254)<br>b) Anisaldehyd-Schwefelsäurereagens (eine frisch bereitete Lösung aus 50 ml Essigsäure und 0,5 ml Anisaldehyd unter Zusatz von 1 ml konz. $H_2SO_4$) |

Für die Untersuchung auf den Apigeningehalt sowie den Apigenin-7-glucosidgehalt mit Hilfe der Hochdruckflüssigkeitschromatographie (HPLC) wurde der Rest der Kamillenblüte unter Rückfluß mit Methanol extrahiert. Der Extrakt wurde an einer $C_{18}$-Umkehrphasensäule chromatographiert.

Anhand dieser Dünnschicht- und HPLC-Chromatogramme konnten die Pflanzen selektiert werden. Überraschenderweise konnten in Populationen von etwa 100 Kamillenpflanzen ein oder zwei Individuen gefunden werden, die einerseits einen unerwartet hohen Anteil an Bisabolol und En-In-Dicycloether enthielten und andererseits auch einen unerwartet hohen Anteil an Apigenin aufwiesen. Zudem blieb bei diesen Pflanzen der Azulengehalt, der bei der Ausgangssorte bereits hoch war, unverändert hoch, während der Gehalt an unerwünschten Bisabololoxiden niedrig war. Die entsprechenden Pflanzen wurden dann zur Samengewinnung von den anderen Kamillenpflanzen getrennt aufbewahrt und vermehrt. Überraschenderweise waren auch in der nächsten Generation einige Individuen mit dem oben beschriebenen Inhaltsstoffmuster vorhanden. Auf diese Weise gelang es in wenigen Schritten, die erfindungsgemäße Kamillensorte zu züchten.

Die erfindungsgemäße Kamille zeichnet sich durch folgendes Inhaltsstoffmuster aus:

| | |
|---|---|
| (-)-α-Bisabolol | > 150 mg/100 g Kamillenblüten |
| Cis-En-In-Dicycloether | > 200 mg/100 g Kamillenblüten |
| Bisabololoxide A + B | < 50 mg/100 g Kamillenblüten |
| Chamazulen | > 100 mg/100 g Kamillenblüten |
| Apigenin | > 20 mg/100 g Kamillenblüten |
| Apigenin-7-glucosid | > 150 mg/100 g Kamillenblüten |

Diese Kamille zeichnet sich nicht nur durch einen hohen Apigeningehalt aus, sondern das Verhältnis Apigenin/ Apigenin-7-glucosid ist auch größer als bei den bisher bekannten Kamillen.

Aus der neuen Kamille hergestellte Extraktpräparate enthalten daher einen deutlich höheren Anteil des muskulotropspasmolytisch wirksamen Apigenins als die bekannten Präparate.

Die Chromosomenzahl der neuen Kamillensorte Robumille wurde wie folgt überprüft:
Die Überprüfung der Polyploidiestufe wurde im Rosettenstadium an Wurzelspitzen im Frühjahr durchgeführt. Vor der Entnahme von 1 cm langen Wurzelspitzen wurden die getopften Pflanzen für 24 h kühl gestellt.

Vorbereitung der Wurzelspitzen:

Die frisch geernteten Wurzelspitzen wurden 6 h in eine 8-Hydroxychinolinlösung (Reagenz 1) gelegt, anschließend in eine warme 1 n HCl (Reagenz 2) überführt und 15 Min. hydrolysiert. Nach der Hydrolyse wurden die Wurzelspitzen in Leitungswasser umgelegt, in welchem sie über einige Tage im Kühlschrank aufbewahrt werden konnte.

Untersuchung der Wurzelspitzen (Quetschmethode) :

Die Wurzelspitze wurde auf einen Objektträger gelegt und ein 0,5 bis 11 mm langes Wurzelspitzchen mit einer Pinzette abgequetscht. Nach Absaugen des Wassers wurden 1 bis 2 Tropfen Orcein-Essigsäure (Reagenz 3,Gebrauchslösung) zugegeben und eine transparente Astrolon M-50 Kunststoffolie 0,25 mm pol/pol (Fa. Dynamit-Nobel AG, Troisdorf) aufgelegt. Das Präparat wurde unter der Folie so lange gequetscht, bis alle Zellen, ohne zu platzen, zu einer einzelligen Schicht auseinandergewichen waren. Für die anschließende mikroskopische Untersuchung wurde ein Tropfen Immersionsöl (Merck) auf die Folie aufgetragen und ein Immersionsobjektiv 100 x verwendet.

Reagenz 1: 0,002 n 8-Hydroxychinolin-Lösung, 72 mg 8-Hydroxychinolin ("Oxin") (Merck) werden in 250 ml dest. Wasser gelöst

Reagenz 2: 10 ml n HCl werden im Trockenschrank auf 60°C erwärmt

Reagenz 3: 2 g Orcein (Merck) werden mit 100 ml Eisessig versetzt und 20 Min. unter Rückfluß gekocht. Nach dem Erkalten wird die Lösung abfiltriert, kühl und dunkel aufbewahrt (Stammlösung). 5 ml der Stammlösung werden mit 5 ml dest. Wasser verdünnt (Gebrauchslösung).

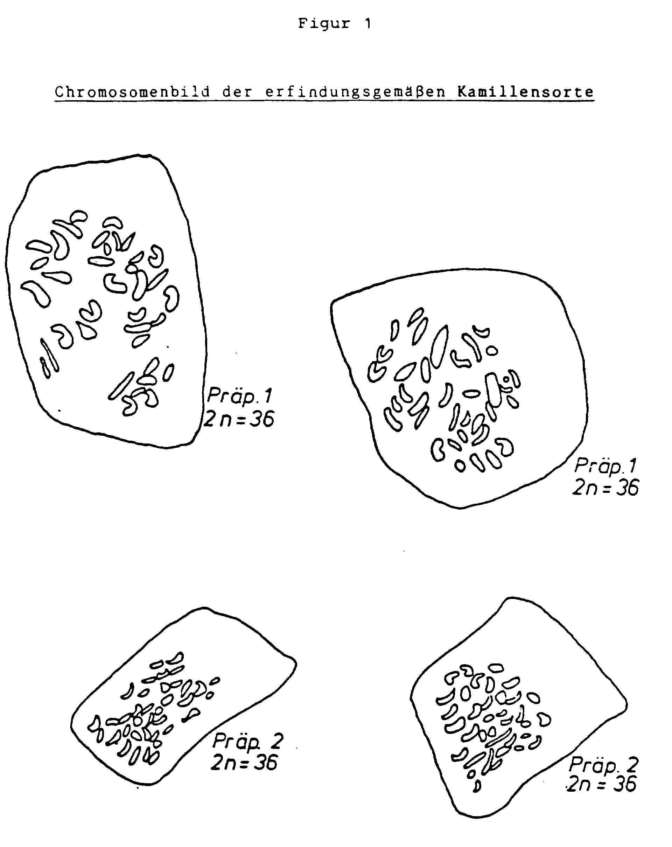

Das Chromosomenbild ist in der Fig. 1 gezeigt.
Zur nachstehend beschriebenen Gewinnung des etherischen Öls wurden die Kamillenblüten in einem Stadium geerntet, in dem 2/3 aller Blüten aufgeblüht waren. Sie enthalten mindestens 0,6 % (V/m = Vol./Masse gemäß DAB 9) blaues etherisches Öl.
Die Blütenköpfchen (Infloreszenzen) wurden bei 30°C bis 50°C bis zu einem Restfeuchtigkeitsgehalt von 5 bis 10% getrocknet.
Eine Samenprobe dieser Kamillensorte ist seit dem 01.12.1987 unter dem Aktenzeichen R 4345 im Botanischen Garten der Philipps-Universität Marburg eingelagert.

Gewinnung des etherischen Öls

Als Ausgangsmaterial dient eine Droge der neuen Kamillensorte Robumille.
2,00 g grob gepulverte Blütenköpfchen werden in einem 250 ml Rundkolben mit 100 ml Wasser versetzt und 2 h einer Rücklaufdestillation unterzogen. Die Meßkapillare dieser Apparatur wird mit Wasser gefüllt und 1 ml Pentan wird dazupipettiert. Die Heizquelle wird während der 2-stündigen Destillation so eingestellt, daß pro Min. ca. 40 Tropfen in den Kolben zurückfließen. Nach Beendigung der Destillation überführt man die Pentanphase in ein Probengläschen. Die Meßkapillare wird mit 2 ml Pentan nachgespült. Zu den vereinigten Pentanphasen gibt man ca. 200 mg getrocknetes Natriumsulfat, schüttelt und läßt die Lösung 30 Min. stehen. Dann wird die Lösung durch eine $G_4$-Nutsche abfiltriert, wobei Reagenzglas und Nutsche mit Pentan nachgespült werden. Nach Abdunsten des Pentans bei 40°C im Wasserbad und nach Trocknen im Exsikkator wird das Gewicht des Öls ermittelt. Der Mittelwert von 10 Gehaltsbestimmungen liegt bei 1 % (V/m).

Gehaltsbestimmung:

1) (-)-α-Bisabolol
Bisabololoxide A und B
Cis-En-In-Ether

Zur Probenvorbereitung setzt man 10,0 g Kamillenblüten mit 50 g Dichlormethan R (Ph. Eur.) an und läßt

unter häufigem Schütteln einen Tag stehen. Anschließend preßt man ab und läßt den Extrakt ca. 2 h zum Absitzen der eventuell vorhandenen Schwebstoffe ruhig stehen.

10,0 ml des Extraktes versetzt man in einem 50 ml-Rundkolben mit 2,0 ml einer 0,1%-igen isopropanolischen Phenanthrenstandardlösung und engt im Rotationsverdampfer bei 30°C im Vakuum zur Trockene ein. Den Rückstand nimmt man mit 5 ml Dichlormethan R (Ph.Eur.) auf. Man chromatographiert dann bei folgenden Bedingungen:

| | |
|---|---|
| Gerät: | Sigma 3 B, Perkin Elmer |
| Einspritzmenge: | 1 µl Probelösung |
| Säule: | 25 m OV 101 Fused silica Kapillare 0,23 mm i.D. |
| Trägergas: | $N_2$, 70 kPa 0,9 ml/min |
| Split: | 1:90 |
| Säulentemperatur: | 150°C 1 Min. |
| | 4°C / Min. |
| | 200°C 17 Min. |
| Analysenzeit: | 30 Min. |
| Detektor: | FID 250°C Range x 1 |
| D | $H_2$ : 170 kPa |
| | Luft: 190 kPa |
| Injektor: | 250°C |

Der Gehalt pro 100 g Kamillenblüten berechnet sich wie folgt:

$$G = \frac{A \cdot 100}{20 \cdot 1{,}33}$$

A = mg/100 ml Auszug
1,33 = Dichte von Dichlormethan

Im Gaschromatogramm erscheinen die Peaks von (-)-α-Bisabolol, Bisabololoxid A und B und Cis- und Trans-En-In-Ether. Das Gaschromatogramm ist frei von Fremdpeaks.

2. Azulen

In einer Apparatur zur Bestimmung des etherischen Öles in Drogen (Ph.Eur. III) versetzt man 10 g Kamillenblüten mit 300 ml Wasser, 3 g Ascorbinsäure R (Ph.Eur.), 15 g 4%iger Natronlauge sowie einigen Siedesteinchen.

Den Ölabscheidungsraum der Apparatur füllt man erst mit Wasser und gibt dann 2 ml Dekalin R (Ph.Eur.) zu.

Nach 2-stündiger Destillation läßt man das Wasser ab und fängt das Dekalin in einem graduierten Reagenzglas auf. Man spült den Ölabscheidungsraum einmal mit Dekalin.

Man versetzt das Reagenzglas mit Dekalin, bis die organische Phase 10 ml ausmacht. Zum Entfernen des Wassers fügt man einige Spatelspitzen getrocknetes Natriumsulfat R (Ph.Eur.) zu, schüttelt und filtriert. Man mißt die Extinktion des Filtrats bei 578 nm im Filterfotometer. Man bestimmt den Gehalt an Azulen pro Einwaage mit Hilfe einer Eichgeraden.

3. Apigenin und Apigenin-7-glucosid

Die Gehaltsbestimmung erfolgte mittels HPLC. Zur Herstellung einer Eichlösung wurden jeweils 3 mg Apigenin und 5 mg Apigenin-7-glucosid genau eingewogen und in Methanol gelöst. Das Methanol wurde in einen Meßkolben gegeben, der auf 100 ml aufgefüllt wurde. Zur Herstellung einer Probenlösung wurden 1 g Kamillenblüten, genau gewogen, unter Rückfluß 1,5 h mit Methanol extrahiert. Die Lösung wurde durch ein Papierfilter filtriert und im Meßkolben mit 100% Methanol auf 100 ml aufgefüllt. Danach wurde durch ein 1µ Filter filtriert.

Die Bedingungen für die HPLC-Chromatographie waren die folgenden:

| | | |
|---|---|---|
| Säule: | reversed phase C18, 10µ | |
| | Hibar 250-4 Merck | |
| Mobile Phase: | A: 3% Essigsäure | |
| | B: Methanol-Tetrahydrofuran-Acetonitril-Wasser-Essigsäure (50:29:325:96:15); | |
| Gradient: | Zeit 0 | 20% B |

| | |
|---|---|
| Zeit 10 | 40% B |
| Zeit 20 | 70% B |
| Zeit 25 | 70% B |
| Zeit 35 | 20% B |

| | |
|---|---|
| Durchfluß: | 1,0 ml/min. |
| Detektion: | 340 nm |
| | Absorptionsbereich 0,05 |
| | Bandbreite 16 |
| | Zeitkonstante: normal |
| Probenmenge: | 10 µl (Schleife) |

Die Auswertung der Analysen erfolgte über die Peakhöhe. Einige Mittelwerte von jeweils 10 Bestimmungen sind nachfolgend in mg/100 g Droge angegeben:

| | Apigenin mg % | Apigenin-7-glucosid mg % | Apigenin Apigenin-7-glucosid % |
|---|---|---|---|
| 1 | 65 | 261 | 25 |
| 2 | 42 | 210 | 20 |
| 3 | 86 | 368 | 23,4 |
| 4 | 61 | 221 | 27,6 |

Aus der erfindungsgemäßen neuen Kamillenprobe können in an sich bekannter Weise Kamillenextrakte, Kamillenöl oder pharmazeutische Mittel hergestellt werden. Für die Extraktion verwendet man beispielsweise gerade oder verzweigte aliphatische Alkohole mit 1 bis 6 Kohlenstoffatomen, wie beispielsweise Methanol, Äthanol, Propanol-(1), Propanol-(2), n-Butanol, iso-Butanol oder Tert-Butanol, Glycerin, Isopropyliden-Glycerol oder Gemische dieser Alkohole mit Wasser. Die Extraktion kann auch mit 2,2-Dimethyl-4-Hydroxymethyl-1,3-Dioxolan oder mit überkritischen Gasen, z.B. mit flüssigem Kohlendioxyd, erfolgen.
Man verwendet im allgemeinen 3 bis 10 Gew.-Teile Lösungsmittel auf einen Gew.-Teil trockene Droge. In die Extraktion können aber auch frische oder gefrorene Kamillenblüten eingesetzt werden. Die Lösungsmittelmenge ist dann entsprechend geringer.

Für die Herstellung von Vollextrakten verwendet man vorteilhafterweise Alkohol/Wasser-Gemische mit einem Wassergehalt von 1 - 80, vorzugsweise 20 - 60, insbesondere 30 - 55 Volumen %.

Die Kamillenflavonoide verhalten sich bei der Extraktion deutlich anders als die anderen Inhaltsstoffe der Kamille. Die Flavonoide lassen sich z.B. mit Methanol wesentlich besser extrahieren als mit Äthanol, letzteres wiederum hat eine größere Extraktionskraft als Isopropanol. Die Ausbeute an Gesamtkamillen-flavonoide (Apigenin, Apigenin-7-glucosid und dessen Acetylderivate) ist - wie oben gesagt - einerseits abhängig von der Art des zur Extraktion verwendeten Alkoholes und andererseits von der Menge des darin enthaltenen Wassers. Ein Ausbeutemaximum an Gesamtflavonoiden liegt bei einem Methanol/Wasser-Gemisch von 80 % (V/V) bzw. bei einem Äthanol/Wasser-Gemisch von 60 % (V/V) bzw. bei einem Isopropanol/Wasser-Gemisch von 50 % (V/V).
Höhere Wasseranteile in den vorbezeichneten Alkohol/Wassergemischen bedingen vermutlich eine geringere Löslichkeit der Flavonoide, andererseits bleiben aber bei höherem Wasseranteil hydrolytische Enzyme aktiv, die in den Kamillenblüten enthalten sind. Dies bewirkt einen Abfall der Glucosid-Konzentration und einen Anstieg des Apigenin-Gehaltes. Man kann daher den Gehalt an freiem Apigenin auch dadurch erhöhen, daß man frische oder nicht allzu intensiv getrocknete und vor allem nicht über 50° erwärmte Kamillendroge beispielsweise mit einem Methanol/Wasser- oder Äthanol/Wasser-Gemisch, das 60 - 70 Vol. % Wasser enthält, mazeriert (3 Tage lang). Die Ausbeute an Apigenin beträgt dann im Falle der Methanol/Wasser-Extraktion bis zu 290 mg/100 g Droge und im Falle von Äthanol/Wasser bis zu 230 mg/100 g Droge.

Will man enzymatische Veränderungen der Zusammensetzung eines Extraktes vermeiden, so ist es empfehlenswert, bei der Extraktion mit Äthanol/Wasser-Gemischen einen Äthanolgehalt von höher als 50 % einzuhalten. Damit wird weitgehend vermieden, daß das Enzym mit in Lösung geht und Stabilitätsprobleme bei den fertigen Präparaten verursacht. Die hydrolytisch wirksamen Enzyme können aber auch durch kurzzeitiges Erhitzen der Droge auf 70 - 90° desaktiviert werden.

Die Herstellung eines ätherischen öles erfolgt ebenfalls in üblicher Weise, z.B. dadurch, daß man die Droge mit Wasser in Gegenwart von ein wenig Ascorbinsäure oder Natrium-Ascorbat bei einem pH-Wert zwischen 4 und 6, vorzugsweise 5 - 5,5 zum Sieden erhitzt. Die Einstellung des pH-Wertes kann z.B. mit Salzsäure erfolgen. Auf ein Gew.-Teil trockene Droge werden beispielsweise 10 - 50 Gew.-Teile Wasser und 0,1 - 1 Gew.-Teil Ascorbinsäure verwendet.

Das aufgefangene wässrige Destillat wird mehrmals mit einem niederen aliphatischen Kohlenwasserstoff, beispielsweise Petroläther oder Pentan, ausgeschüttelt. Man trocknet die organische Phase und entfernt das Lösungsmittel bei vermindertem Druck.

Das so gewonnene Kamillenöl wird beispielsweise in reiner Form oder als Lösung in einem physiologisch verträglichem Lösungsmittel für die Herstellung von pharmazeutischen Präparaten verwendet. Es kann auch wässrig alkoholischen Kamillenextrakten beigemischt werden, um diese auf einen bestimmten Wirkstoffgehalt einzustellen.

Beispiel 1:

Herstellung eines äthanolischen Extraktes.

500 g der erfindungsgemäßen Kamillenprobe (getrocknet bei 30 - 50°C) werden in einem Mischgefäß mit 2,5 l wässrigem Äthanol (60 Vol.% Äthanol) 3 Tage mazeriert. Die Droge wird anschließend abgepreßt, die Lösung filtriert, der Wirkstoffgehalt in der oben beschriebenen Weise ermittelt. Der Gehalt an Apigenin-7-Glucosid beträgt 547 mg % und an Apigenin 92 mmg %.

Beispiel 2:

Herstellung eines ätherischen Kamillenöles.

200 g erfindungsgemäße Kamillendroge (Kamillenblüten, getrocknet bei 30 - 50°C im Schatten) werden in einem 5 1-Rundkolben mit 2,5 l Wasser und 2 g Natriumascorbat vermischt und mit 1 NHCl wird ein pH-Wert von 5,0 eingestellt. Innerhalb von 2,5 - 3 Stunden werden etwa 1 - 1,5 1 Destillat aufgefangen. Letzteres wird dreimal mit je 75 ml Petroläther ausgeschüttelt. Die vereinigten Petrolätherfraktionen werden mit wasserfreiem Natriumsulfat getrocknet, das Lösungsmittel wird anschließend bei reduziertem Druck entfernt. Die Ausbeutean ätherischem Öl beträgt 1,6 g.

## Patentansprüche

1. Kamillendroge der Kulturpflanzenart echte Kamille (Matricaria chamomilla L) mit

   einem Chamazulengehalt von 100 bis 250 mg,

   einem (-)-$\alpha$-Bisabololgehalt von 150 bis 300 mg,

   einem Cis-En-In-Dicycloethergehalt von 200 bis 350 mg,

   einem Gehalt an freiem Apigenin von 20 bis 300 mg, und

   einem Gehalt an Apigenin-7-glucosid von 150 bis 700 mg,

   bezogen auf 100 g bei 30° bis 50°C bis zu einem Restfeuchtegehalt von 5 bis 10% getrocknete Kamillenblüten,

2. Kamillendroge nach Anspruch 1 mit einem Chamazulengehalt von 100 bis 200 mg und insbesondere von 100 bis 160 mg.

3. Kamillendroge nach Anspruch 1 oder 2 mit einem (-)-$\alpha$-Bisabololgehalt von 170 bis 250 mg und insbesondere von 170 bis 230 mg.

4. Kamillendroge nach einem der Ansprüche 1 bis 3 mit einem Cis-En-In -Dicyloethergehalt von 200 bis 300 mg und insbesondere von 200 bis 250 mg.

5. Kamillendroge nach einem der vorhergehenden Ansprüche mit einem Gehalt an freiem Apigenin von 40 bis 120 mg und insbesondere von 50 bis 100 mg.

6. Kamillendroge nach einem der vorhergehenden Ansprüche mit einem Gehalt an Apigenin-7-glucosid von 200 bis

600 mg und insbesondere von 250 bis 450 mg.

**7.** Verwendung der Kamillendroge nach einem der Ansprüche 1 bis 6 zur Herstellung von Kamillenextrakten, Kamillenöl und pharmazeutischen Mitteln.

## Claims

**1.** Chamomile drug from the cultivated plant variety true chamomile (Matricaria chamomilla L) having

- a chamazulene content of 100 to 250 mg,
- an (-)-α-bisabolol content of 150 to 300 mg,
- a cis-en-in-dicycloether content of 200 to 350 mg,
- a content of free apigenin of 20 to 300 mg, and
- a content of apigenin-7-glucoside of 150 to 700 mg,

based on 100 g of camomile flowers dried at 30° to 50°C to a residual moisture content of 5 to 10%.

**2.** Chamomile drug according to claim 1, with a chamazulene content of 100 to 200 mg and particularly 100 to 160 mg.

**3.** Chamomile drug according to claim 1 or 2, with an (-)-α-bisabolol content of 170 to 250 mg and particularly 170 to 230 mg.

**4.** Chamomile drug according to one of claims 1 to 3, with a cis-en-in-dicycloether content of 200 to 300 mg and particularly 200 to 250 mg.

**5.** Chamomile drug according to one of the preceding claims, with a content of free apigenin of 40 to 120 mg and particularly 50 to 100 mg.

**6.** Chamomile drug according to one of the preceding claims, with a content of apigenin-7-glucoside of 200 to 600 mg and particularly 250 to 450 mg.

**7.** Use of the chamomile drug according to one of claims 1 to 6 for the preparation of chamomile extracts, chamomile oil and pharmaceutical agents.

## Revendications

**1.** Drogue de camomille tiré de la variété de plante cultivée camomille véritable (Matricaria chamomilla L) présentant:

une teneur en chamazulène de 100 à 250 mg,
une teneur en (-)-α-bisabolol de 150 à 300 mg,
une teneur en cis-ène-yne-dicycloéther de 200 à 350 mg,
une teneur en apigénine libre de 20 à 300 mg, et
une teneur en apigénine-7-glucoside de 150 à 700 mg,

pour 100 g de fleurs de camomille séchées à 30° à 50°C jusqu'à une teneur en humidité résiduelle de 5 à 10 pour cent.

**2.** Drogue de camomille selon la revendication 1 présentant une teneur en chamazulène de 100 à 200 mg et en particulier de 100 à 160 mg.

**3.** Drogue de camomille selon la revendication 1 ou 2, présentant une teneur en (-)-α-bisabolol de 170 à 250 mg et en particulier de 170 à 230 mg.

**4.** Drogue de camomille selon l'une quelconque des revendications 1 à 3, présentant une teneur en cis-ène-yne-dicycloéther de 200 à 300 mg, et en particulier de 200 à 250 mg.

**5.** Drogue de camomille selon l'une quelconque des revendications précédentes, présentant une teneur en apigénine libre de 40 à 120 mg et en particulier de 50 à 100 mg.

**6.** Drogue de camomille selon l'une quelconque des revendications précédentes présentant une teneur en apigénine-7-glucoside de 200 à 600 mg et en particulier de 250 à 450 mg.

**7.** Utilisation de la drogue de camomille selon l'une quelconque des revendications 1 à 6 pour la préparation d'extraits de camomille, d'huile de camomille et de remèdes pharmaceutiques.

Figur 1

Chromosomenbild der erfindungsgemäßen Kamillensorte